(19) Europäisches Patentamt
European Patent Office
Office européen des brevets

(11) **EP 2 129 296 B1**

(12) **FASCICULE DE BREVET EUROPEEN**

(45) Date de publication et mention de la délivrance du brevet:
**20.05.2015 Bulletin 2015/21**

(21) Numéro de dépôt: **08787823.7**

(22) Date de dépôt: **20.03.2008**

(51) Int Cl.:
*A61B 8/08* (2006.01)     *G01S 7/52* (2006.01)

(86) Numéro de dépôt international:
**PCT/FR2008/000374**

(87) Numéro de publication internationale:
**WO 2008/135659 (13.11.2008 Gazette 2008/46)**

(54) **DISPOSITIF POUR MESURER DES PROPRIÉTÉS VISCOÉLASTIQUES DE TISSUS BIOLOGIQUES ET PROCÉDÉ UTILISANT CE DISPOSITIF.**

VORRICHTUNG ZUR MESSUNG DER VISKOELASTIZITÄT BIOLOGISCHER GEWEBE UND VERFAHREN ZUR VERWENDUNG DIESER VORRICHTUNG

DEVICE FOR MEASURING THE VISCOUS-ELASTIC PROPERTIES OF BIOLOGICAL TISSUES AND METHOD USING SAID DEVICE

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **21.03.2007 FR 0702050**

(43) Date de publication de la demande:
**09.12.2009 Bulletin 2009/50**

(73) Titulaire: **Echosens**
**75013 Paris (FR)**

(72) Inventeurs:
• **SANDRIN, Laurent**
**F-92240 l'Hay-les-Roses (FR)**
• **YON, Sylvain**
**F-92260 Fontenay aux roses (FR)**

(74) Mandataire: **Lebkiri, Alexandre**
**Cabinet Camus Lebkiri**
**25, Rue de Maubeuge**
**75009 Paris (FR)**

(56) Documents cités:
**EP-A- 1 040 789     EP-A- 1 623 675**
**FR-A- 2 844 058     US-A1- 2004 167 403**

**Description**

L'invention se rapporte à un dispositif mesurant des propriétés viscoélastiques de tissus biologiques et à un procédé utilisant ce dispositif.

L'élastographie effectue une mesure non invasive de propriétés viscoélastiques, dénommées PV par la suite, de tissus biologiques afin de permettre le diagnostic, le dépistage ou le suivi de traitements relatifs à, par exemple, des organes tels que le foie, la peau ou des vaisseaux sanguins.

Un tel procédé est décrit, par exemple, dans la demande de brevet FR 2869521, déposée le 3 mai 2004 au nom de la société Echosens Société Anonyme.

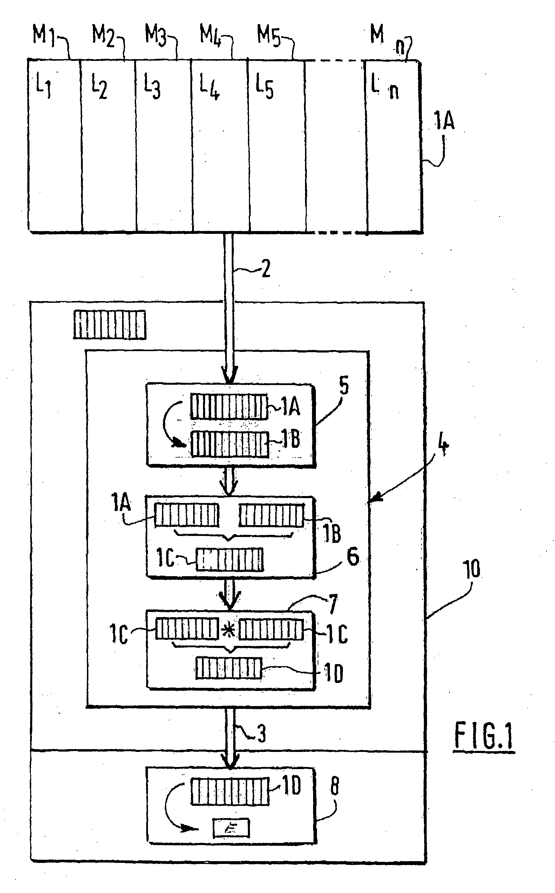

En référence à la figure 1, le fonctionnement d'un dispositif 10 mettant en oeuvre ce procédé peut se réaliser en trois étapes :

- Une première étape au cours de laquelle le dispositif 10 acquiert et mémorise des données ultrasonores dans une mémoire.

Pour cela, des émissions d'ondes ultrasonores sont effectuées dans les tissus observés au moyen d'un ou de plusieurs transducteurs.

L'émission de chaque onde ultrasonore, dénommée tir par la suite, engendre des ondes ultrasonores réfléchies au fur et à mesure que l'onde ultrasonore émise se propage dans des tissus biologiques comprenant des particules diffusantes.

Des données relatives aux ondes ultrasonores réfléchies peuvent être recueillies par le dispositif 10, les données d'ondes ultrasonores réfléchies lors d'un tir i formant une ligne ultrasonore $L_i$ mémorisée dans une mémoire dédiée Mi.

L'ensemble de n lignes ultrasonores $L_1$, $L_2$, $L_3$, $L_4$, $L_5$ ... $L_n$ propres à une mesure de PV comprenant une séquence de tirs T1, T2, T3, T4, T5, ... Tn est dénommé une acquisition 1A mémorisée à l'aide des mémoires M1, M2, M3, M4, M5, ...Mn.

- Une seconde étape 2 au cours de laquelle l'acquisition 1A est transférée vers un premier calculateur 5. Un tel transfert permet de traiter l'acquisition 1A à l'aide de composants dédiés aux calculs décrits ultérieurement.
- Une troisième étape 3 de transfert des résultats des calculs du traitement pour aboutir aux valeurs E de PV des tissus biologiques observés.

Pour obtenir ces valeurs E, le dispositif 10 doit effectuer une série 4 de calculs intermédiaires visant à obtenir un tableau 1D de déplacements des tissus observés en fonction de leur distance vis-à-vis d'un transducteur effectuant l'émission - le transducteur étant animé d'un mouvement de basse fréquence inférieure à 500 Hz - et/ou la réception d'ondes ultrasonores. Cette distance est également dénommée « profondeur » par la suite.

Pour cela, le premier calculateur 5 détermine un tableau 1B de paramètres de déplacements relatifs du transducteur par rapport aux tissus observés.

Puis un calculateur 6 utilise ce tableau 1B de paramètres de déplacements relatifs pour corriger l'acquisition 1A et obtenir une acquisition corrigée 1C compensée des déplacements relatifs du transducteur.

Finalement, un calculateur 7 détermine, sur la base de l'acquisition corrigée 1C, le tableau 1D des déplacements tissulaires propres aux tissus observés. Ce déplacement des tissus est dénommé par la suite déplacement intrinsèque.

Cette opération peut être effectuée par une technique dite d'autocorrélation, d'intercorrélation ou plus généralement toute technique d'estimation de déplacement à partir de signaux ultrasonores.

ii) A partir de ce tableau 1D de déplacements intrinsèques, une mesure E de PV des tissus observés peut être déterminée par un calculateur 8.

L'invention résulte de la constatation qu'un tel dispositif 10 présente des inconvénients. Notamment, ce dispositif requiert de nombreuses mémoires Mi, de taille relativement importante, pour mémoriser une acquisition 1A, un tableau 1B de paramètres de déplacements relatifs, une acquisition corrigée 1C et/ou un tableau 1D de déplacements intrinsèques.

Or, le coût et la taille de ces mémoires sont élevés, notamment du fait qu'elles requièrent des moyens pour traiter des débits élevés de donnés - plusieurs Mo/s. Ainsi, le coût et l'encombrement d'un dispositif 10 équipé de ces mémoires sont accrus proportionnellement.

En outre, de nombreux transferts de données doivent être gérés vers/dans le dispositif 10. De ce fait, le dispositif 10 comprend de nombreux moyens de transferts de données qui augmentent à nouveau son coût et son encombrement.

Par ailleurs, ces calculs et ces transferts imposent un délai important entre l'émission d'une séquence de tirs ultrasonores et l'obtention d'une mesure de PV associée.

Par exemple, la mémorisation d'une acquisition 1A requiert typiquement une durée de l'ordre de 100 ms pour une profondeur maximum de l'ordre de 100 mm en élastographie impulsionnelle à une dimension.

En outre, la durée nécessaire pour transférer des données et effectuer les différents calculs requis au sein du dispositif 10 atteint plusieurs secondes en considérant un seul axe de profondeur.

Finalement, les données à transférer sont groupées et présentent typiquement des volumes de plusieurs mégaoctets. Un transfert rapide de ces groupes de données requiert l'emploi de liaison haut débit dont le coût est important tandis que l'emploi de liaisons de débit inférieur accroît les délais de transferts de plusieurs secondes. Typiquement, le transfert 2 d'une acquisition 1A dont la taille est de 4 mégaoctets nécessite 3,4 secondes avec une liaison de 10 Mbps.

L'invention vise à résoudre au moins un des problèmes précédemment indiqués. C'est pourquoi, elle concerne un dispositif destiné à mesurer des PV de tissus biologiques grâce à un traitement d'ondes ultrasonores réfléchies par ces tissus lorsqu'ils sont parcourus par une onde de cisaillement, ce dispositif comprenant des moyens pour :

-   Former une acquisition avec des lignes ultrasonores telles que chaque ligne ultrasonore comprend des données relatives aux ondes ultrasonores générées par réflexion d'un même tir, et
-   Déterminer un paramètre relatif au déplacement d'un transducteur ultrasonore par rapport aux tissus,

ce dispositif étant caractérisé en ce qu'il comprend des moyens pour traiter de premières lignes ultrasonores de l'acquisition, à l'aide du paramètre relatif, avant l'acquisition de secondes lignes ultrasonores de cette même acquisition, afin de déterminer le déplacement intrinsèque des tissus biologiques à partir de ces premières lignes.

Grâce à un tel dispositif, il est possible de réaliser le traitement de lignes ultrasonores au cours d'une acquisition, c'est-à-dire au fur et à mesure de la formation des lignes d'une même acquisition.

Ainsi, le délai de traitement de l'acquisition est fortement réduit car un dispositif conforme à l'invention peut débuter le traitement de lignes d'une acquisition dès que les premières lignes de cette acquisition sont formées.

Pour rappel, le traitement d'une acquisition débute, selon l'art antérieur, lorsque la dernière ligne de cette acquisition est formée.

Grâce à l'invention, de nombreuses lignes d'une acquisition ont été traitées avant que la dernière ligne de cette acquisition ne soit formée. On réduit ainsi considérablement le temps nécessaire pour obtenir une mesure du déplacement intrinsèque des tissus observés.

D'une façon générale, en considérant $\Delta$ (cal) le temps de traitement de deux lignes et n le nombre de lignes d'une acquisition, l'art antérieur effectue un traitement de l'acquisition aboutissant au plus tôt après un délai supérieur à $(n-1)*\Delta(cal)$ à compter de la fin de l'acquisition, les temps de transferts des données étant supposés nuls.

Dans des conditions analogues, un dispositif conforme à l'invention peut finaliser le traitement d'une acquisition après un délai de l'ordre de $\Delta(cal)$ à compter de la fin de l'acquisition.

Un dispositif conforme à l'invention présente également l'avantage de réduire considérablement le nombre de mémoires et de transferts de données nécessaires au traitement des lignes.

En effet, une mémoire de taille limitée peut être mise en oeuvre pour stocker un nombre réduit de lignes puisque seules les lignes en cours de traitement et/ou d'acquisition doivent être mémorisées.

De fait, le stockage des lignes déjà traitées n'est pas nécessaire et la mémoire allouée à de telles lignes déjà traitées peut être libérée et allouée à d'autres lignes.

Dans une réalisation, le dispositif comprend des moyens pour calculer le déplacement intrinsèque à un instant et à une profondeur donnés.

Selon une réalisation, le dispositif comprend des moyens pour déterminer différents déplacements intrinsèques de façon à former au moins un tableau chronologique avec ces déplacements intrinsèques, chaque colonne du tableau étant fonction de la profondeur à laquelle sont mesurés des déplacements intrinsèques.

Dans une réalisation, le dispositif comprend des moyens pour que le temps de calcul d'un déplacement intrinsèque soit inférieur au temps séparant deux tirs ultrasonores successifs.

Selon une réalisation, le dispositif comprend des moyens pour que la mémoire allouée à une première ligne ultrasonore soit rendue disponible, après son traitement, pour mémoriser une ligne ultrasonore ultérieure.

Dans une réalisation, le dispositif comprend des moyens pour que seules deux lignes correspondant à un calcul de déplacement intrinsèque en cours, ainsi qu'une ligne (Ln+2) en cours d'acquisition, soient mémorisées par le dispositif.

Selon une réalisation, le même calculateur effectue de façon simultanée des opérations liées à une formation de ligne ultrasonore et à un calcul de déplacement intrinsèque.

Dans une réalisation, le dispositif comprend des moyens pour que les lignes ultrasonores traitées proviennent d'un unique élément émetteur/récepteur.

Selon une réalisation, le dispositif comprend des moyens pour que des lignes ultrasonores soient formées grâce à une focalisation électronique avec au moins un transducteur comprenant plusieurs éléments.

Dans une réalisation, le dispositif comprend des moyens pour calculer un déplacement relatif du transducteur par rapport aux tissus observés à l'aide d'au moins :

-   une mesure physique externe, telle que la position dudit transducteur par rapport à un référentiel donné,
-   une mise en forme d'une mesure biophysique, tel qu'un signal obtenu à partir d'un rythme cardiaque ou respiratoire, ou
-   un calcul effectué à partir des données ultrasonores.

Dans une réalisation, le dispositif comprend des moyens pour que le déplacement intrinsèque représente ou dérive d'au moins un des éléments suivants : une mesure de déplacement, une vitesse, une vitesse de déformation, une mesure de déformation.

Selon une réalisation, le dispositif comprend des moyens pour traiter en parallèle différentes acquisitions réalisées sur différents axes géométriques.

Dans une réalisation, le dispositif comprend des moyens pour effectuer des mesures de façon continue.

La présente invention concerne également un procédé mettant en oeuvre un dispositif conforme à l'une des réalisations précédentes.

L'invention concerne également une sonde dédiée à la mesure de PV de tissus biologiques par élastographie, caractérisé en ce qu'elle comprend un dispositif conforme à l'une des réalisations précédentes.

D'autres caractéristiques et avantages de l'invention apparaîtront à la lumière de la description d'une réalisation de l'invention effectuée ci-dessous, à titre illustratif et non limitatif, en faisant référence aux figures ci-jointes sur lesquelles :

- la figure 1, déjà décrite, est un schéma fonctionnel d'un dispositif connu de mesure de PV, et
- la figure 2 représente différents délais mis en oeuvre au cours d'une acquisition,
- la figure 3 est un schéma fonctionnel d'un dispositif de mesure de PV conforme à l'invention,
- les figures 4a et 4b sont des schémas fonctionnels d'un dispositif de mesure de PV conforme à une seconde réalisation de l'invention, et
- la figure 5 est un schéma électronique d'un dispositif de mesure de PV selon l'invention.

Un dispositif conforme à l'invention mesure des PV de tissus biologiques par élastographie, à savoir grâce au traitement d'ondes ultrasonores réfléchies par ces tissus lorsqu'ils sont parcourus par une onde de cisaillement.

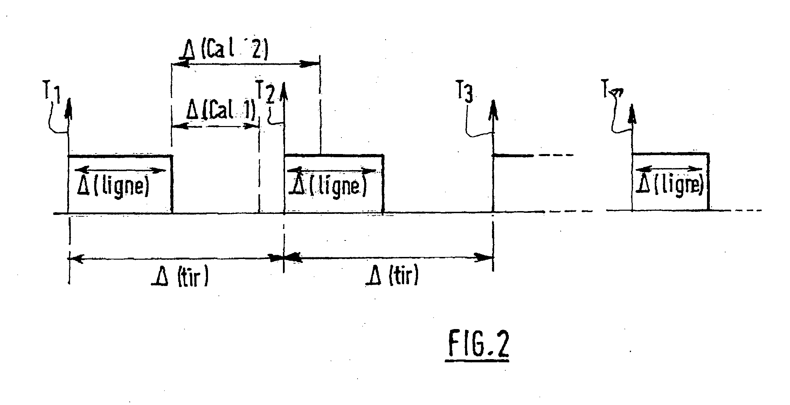

En référence à la figure 2, un tel dispositif utilise des tirs T1, T2, T3, ...Tn d'ondes ultrasonores dont la fréquence est typiquement comprise entre 1 et 10 MHz, et plus généralement entre 0,1 et 40 MHz.

Ces tirs sont effectués en respectant un délai $\Delta$(tir) entre chaque tir compris entre 0,1 ms et 2 ms, plus généralement entre 0,05 ms et 10 ms.

A la suite de chaque tir, des données relatives aux ondes réfléchies par un tir T1, T2, T3, ...Tn servent à former des lignes L1, L2, L3, ...Ln de durée $\Delta$(ligne) comprise entre 50 et 100 $\mu$s, et plus généralement entre 5 et 1000 $\mu$s.

Il convient également de noter que la mesure de PV d'un milieu s'effectue à partir de la mesure d'un paramètre relatif à la propagation d'ondes élastiques de cisaillement - comme la vitesse ou la viscosité de cisaillement - dont la vitesse est typiquement comprise entre 1 et 10 m/s, plus généralement entre 0,1 et 20 m/s.

A titre d'exemple la PV mesurée peut être le module de cisaillement, noté $\mu$, obtenu à partir de la mesure de la vitesse de cisaillement notée Vs en utilisant l'équation suivante :

$$\mu = \rho Vs^2$$

Où $\rho$ est la densité du milieu étudié.

Ces ondes de cisaillement sont engendrées par tout moyen tel qu'un transducteur électrodynamique placé à la surface des tissus, un transducteur ultrasonore utilisé pour déplacer les tissus à distance par pression de radiation ou les mouvements internes aux tissus liés à une activité biophysique comme cardiaque ou respiratoire.

Dans une première variante de l'invention, on considère un dispositif conforme à l'invention comprenant des moyens de calculs et de transfert tels qu'il peut déterminer, à partir d'une acquisition partielle comprenant deux lignes L1 et L2 successivement formées:

- un paramètre relatif au déplacement du transducteur par rapport aux tissus observés,
- une acquisition partielle corrigée, et
- un déplacement intrinsèque,

au cours d'une période de calculs $\Delta$(call) inférieure au délai $\Delta$(tir) entre deux tirs successifs diminué du temps $\Delta$(ligne) nécessaire à la formation d'une ligne.

Dans cette situation, représentée en tirets sur la figure 2, un dispositif conforme à l'invention peut traiter les lignes à l'aide de deux mémoires M1 et M2 telles que chaque mémoire est dédiée à la formation d'une unique ligne.

En répétant ces opérations, un dispositif conforme à l'invention dispose des déplacements intrinsèques de deux premières lignes lorsque des secondes lignes sont formées.

De fait, le contenu de la mémoire L1 peut être remplacé par les données propres à une nouvelle ligne L3 ultérieurement formée par rapport aux lignes L1 et L2 traitées. Ainsi, on peut déterminer pour les lignes L2 et L3, de façon analogue aux lignes L1 et L2 :

- un second paramètre relatif au déplacement,
- une seconde acquisition partielle corrigée, et
- un second déplacement intrinsèque.

Comme déjà indiqué, une telle opération permet un gain de temps important et requiert des moyens réduits de traitement et de transferts des données puisque les lignes sont traitées au fur et à mesure de leur formation.

Une seconde variante de l'invention peut être notamment mise en oeuvre lorsque, comme représenté en trait plein sur la figure 2, le temps de calculs $\Delta$(cal2) est supérieur au délai $\Delta$(tir) de tir diminué du temps $\Delta$(ligne) de formation d'une ligne.

Dans ce cas, un dispositif conforme à l'invention peut fonctionner avec trois mémoires M1, M2 et M3 en stockant dans une mémoire une ligne en formation tandis que deux autres mémoires stockent deux lignes déjà formés et en cours de traitement.

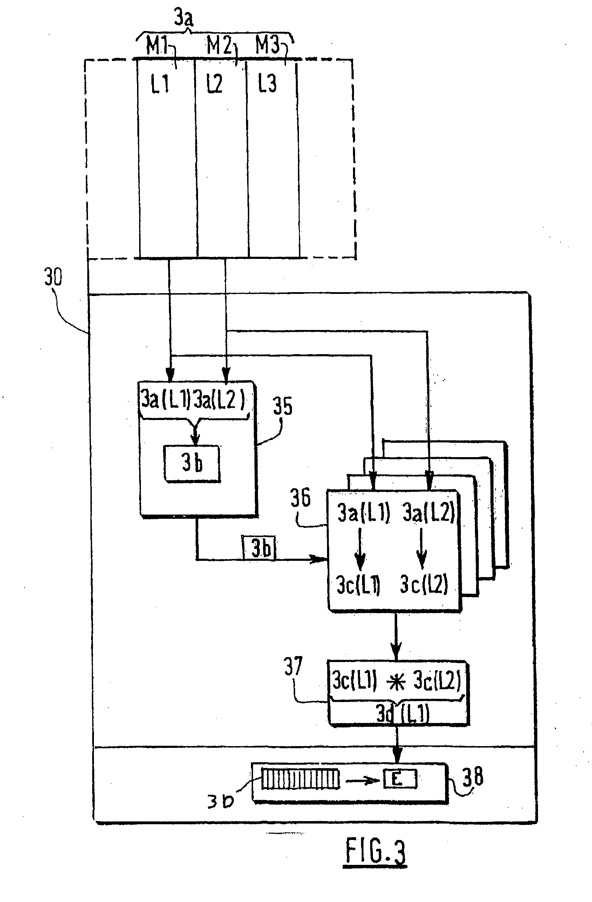

Plus précisément, un tel dispositif 30 - figure 3 - comprend des moyens pour utiliser une acquisition 3a partielle avec des lignes $L_1$, $L_2$ et $L_3$ de données ultra-

sonores.

Il convient de noter que cette acquisition 3a partielle utilise une mémoire de taille réduite par rapport à la mémoire requise pour l'acquisition 1A selon l'art antérieur.

De fait et conformément à l'invention, de premières lignes ultrasonores $L_1$ et $L_2$ sont traitées, au moyen d'un paramètre 3b relatif au déplacement du transducteur vis-à-vis des tissus, avant la formation de secondes lignes de cette même acquisition.

Pour cela, le dispositif 30 comprend un calculateur 35 traitant une acquisition partielle 3a(L1) 3a(L2) limitée à ces premières lignes $L_1$ et $L_2$ en déterminant le paramètre 3b relatif au déplacement entre le transducteur ultrasonore considéré - généralement le transducteur émetteur/récepteur d'ondes ultrasonores - et les tissus observés.

De plus, le dispositif 30 comprend un calculateur 36 déterminant une acquisition partielle corrigée 3c(L1) 3c(L2) permettant d'obtenir, à l'aide d'un calculateur 37, le déplacement intrinsèque 3d(h1) des tissus biologiques à un instant t et à une profondeur z.

A l'aide du traitement successif de lignes par un calculateur 38, différents déplacements intrinsèques 3d(Li) peuvent être obtenus de façon à former progressivement un tableau 3D analogue au tableau 1D formé selon l'art antérieur et représenté figure 1.

Plus précisément, chaque colonne du tableau 3D contient les déplacements intrinsèques mesurés pour un même tir, et donc à un temps donné noté t, en fonction de la profondeur notée z à laquelle est mesuré ce déplacement intrinsèque 3D.

Toutefois, le temps de calcul nécessaire pour obtenir ce tableau est fortement réduit puisqu'il est réalisé au fur et à mesure de la formation et du traitement des lignes.

Par ailleurs, la mémoire utilisée pour stocker des informations relatives à une première ligne ultrasonore $L_1$ déjà traitée peut être rendue disponible.

Dans une réalisation, le même calculateur 36 effectue des opérations simultanées liées à une mémorisation des lignes ultrasonores $L_1$ et $L_2$ et à un calcul du déplacement intrinsèque à partir de ces lignes.

A cet effet, ce calculateur reçoit en parallèle les données relatives à ces lignes $L_1$ et $L_2$, le paramètre 3b relatif au déplacement étant alors déterminé pour ces dernières par le calculateur 35.

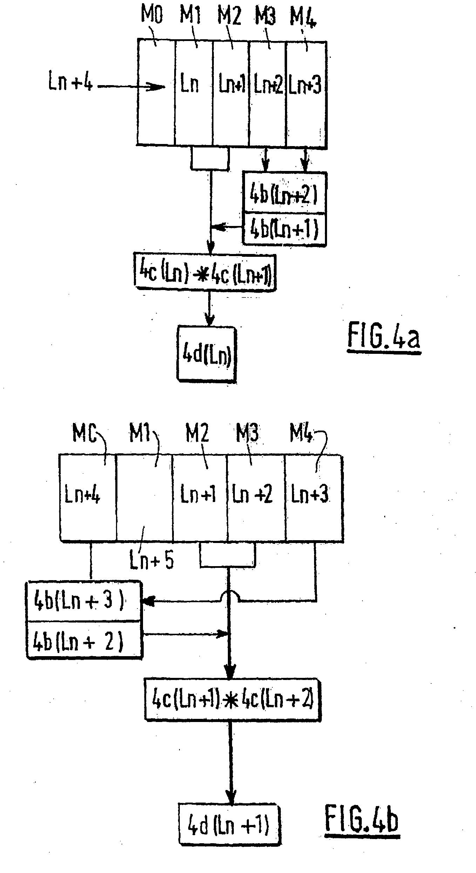

En référence aux figures 4a et 4b est décrit une deuxième mise en oeuvre d'un procédé de traitement de lignes ultrasonores conformément à l'invention.

Selon ce procédé, 5 mémoires M0, M1, M2, M3 et M4 sont mises en oeuvre pour mémoriser des lignes de données Li relatives à une acquisition comprenant m lignes. Ainsi, i varie entre 1 et m.

La figure' 4a illustre l'utilisation des mémoires M0, M1, M2, M3, M4 lorsque la ligne L(n+4) est en cours de formation. A ce stade :

- la mémoire M0 est dédiée à l'acquisition ou formation de la ligne L(n+4).
- les mémoires M1 et M2 sont respectivement dédiées aux lignes Ln et Ln+1. Ces dernières sont en cours de traitement pour déterminer un déplacement intrinsèque 4d(Ln) au moyen d'une acquisition partielle corrigée 4c(Ln) 4c(Ln+1).
- les mémoires M3 et M4 sont respectivement dédiées aux lignes Ln+2 et Ln+3 qui sont en cours de traitement pour déterminer un paramètre 4b(Ln+2) relatif au déplacement.

Il convient de noter que, à ce stade du calcul, le paramètre 4b(Ln+1) relatif au déplacement.a été calculé et mémorisé lors d'étapes précédentes à partir des lignes Ln+1 et Ln+2.

La figure 4b illustre l'utilisation de ces mêmes mémoires M0, M1, M2, M3, M4 lorsque la ligne L(n+5) est en cours de formation. A ce stade, la ligne Ln n'est plus nécessaire et peut être effacée de telle sorte que :

- la mémoire M1 est dédiée à l'acquisition de la ligne L(n+5).
- les mémoires M2 et M3 mémorisent encore les lignes Ln+1 et Ln+2 qui sont désormais utilisées pour déterminer un déplacement intrinsèque 4d(Ln+1) à l'aide du paramètre 4b(Ln+1) de déplacement relatif préalablement calculé et mis en mémoire pour ces deux lignes.
- les mémoires M4 et M0 sont respectivement dédiées aux lignes Ln+3 et Ln+4 qui sont désormais utilisées pour déterminer un paramètre 4b(Ln+4) de déplacement relatif.

A ce stade du calcul, le paramètre relatif au déplacement déterminé à partir des lignes Ln+2 et Ln+3 a été calculé à l'étape précédente et est mis en mémoire.

En résumé, les mémoires M0, M1, M2, M3, M4 sont attribuées de façon successive à la formation d'une ligne, à la mémorisation de cette ligne pour un calcul de paramètre relatif au déplacement ou à la mémorisation de cette ligne pour un calcul de déplacement intrinsèque.

Il convient de noter qu'un dispositif conforme à l'invention présente une grande compacité qui permet son positionnement dans la tête d'une sonde, contrairement au dispositif 10 selon l'art antérieur qui doit être déporté de la sonde sur une unité dédiée.

Indépendamment de la position du dispositif, le transducteur mis en oeuvre pour former des lignes peut présenter un unique ou plusieurs élément(s) destiné(s) à transformer des ondes ultrasonores, réfléchies par les tissus biologiques, en signaux électriques.

Lorsque plusieurs éléments sont utilisés, ces lignes ultrasonores peuvent être formées grâce à une focalisation électronique obtenue par une formation de voie avec au moins un transducteur comprenant plusieurs éléments.

Par ailleurs, le déplacement relatif du transducteur vis-à-vis des tissus observés est obtenu par un calcul effectué à partir de données ultrasonores dans la réalisation décrite.

Toutefois, ce déplacement relatif peut également être déterminé au moyen d'une mesure physique externe, telle que la position dudit transducteur par rapport à un référentiel donné, ou par la mise en forme d'une mesure biophysique, tel qu'un signal obtenu à partir d'un rythme cardiaque ou respiratoire.

Différentes variantes de l'invention sont également possible en déterminant le déplacement intrinsèque du tissus en fonction de divers paramètres, tels qu'au moins un des paramètres suivants ou une dérivé d'un tel paramètre: une mesure de déplacement, une vitesse, une vitesse de déformation, une mesure de déformation.

De façon analogue, l'invention peut être mise en oeuvre en traitant par des systèmes parallèles des lignes propres à une même acquisition conformément à l'invention. Dans ce cas, différents paramètres relatifs au déplacement peuvent être obtenus simultanément afin d'obtenir un déplacement intrinsèque 3d, comme montré sur la figure 3.

La réduction du temps d'acquisition est telle qu'il est possible d'obtenir une mesure des PV de façon pratiquement simultanée à l'émission du dernier tir.

Une telle rapidité accroît le confort d'utilisation du dispositif et permet la mise en place de différents modes d'utilisation. Par exemple, un mode d'utilisation « séquentiel », tel que le dispositif effectue un nombre limité d'acquisition, ou un mode d'utilisation « continu » ou « temps réel », tel que le dispositif mesure de façon permanente une valeur des PV des tissus observés.

Avantageusement, le dispositif comprend un afficheur sur lequel est affichée la PV du milieu. Cette dernière peut être mise à jour au fur et à mesure de la réalisation des acquisitions sous de nombreuses formes, comme par exemple en conservant la valeur instantanée, en conservant la valeur médiane ou la valeur moyenne depuis le début des acquisitions, en conservant la valeur médiane ou la valeur moyenne depuis sur une durée fixe (par exemple 2 secondes).

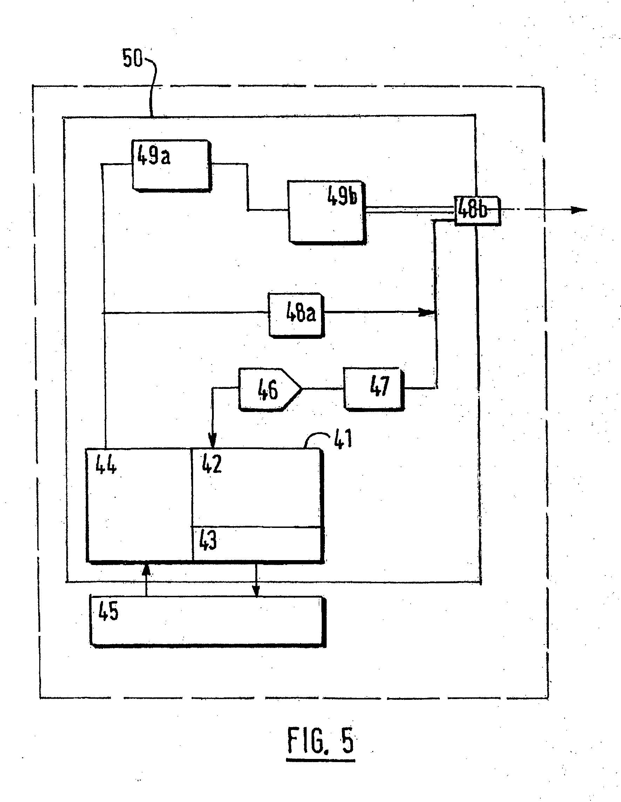

Un schéma d'un dispositif 50 conforme à l'invention est décrit ci-dessous à l'aide de la figure 5.

Le dispositif 50 est composé d'un moteur linéaire 49b permettant le déplacement d'un transducteur ultrasonore mono-élément 48b. Ainsi et comme connu dans l'état de l'art, le transducteur ultrasonore est utilisé tout à la fois comme point de génération d'une onde élastique basse fréquence et comme outil de visualisation de cette onde grâce aux ultrasons. La chaîne électronique inclut également un amplificateur 49a permettant au moteur 49b de disposer de la puissance suffisante pour générer l'onde élastique.

Un amplificateur 48a permet au transducteur 48b de disposer de la puissance suffisante pour générer les ondes acoustiques ultrasonores.

La chaîne électronique comprend également une chaine de préamplification et de filtrage 47 et un convertisseur Analogique-Numérique 46 afin de fournir au calculateur 41 les lignes ultrasonores décrites dans l'invention.

Le calculateur 41 regroupe dans un même composant physique les éléments suivants :

- Un contrôleur de l'acquisition 44, permettant de contrôler les séquences de tirs ultrasonores et d'émission des ondes élastiques.
- Un calculateur de déplacement 42 implémentant l'invention décrite par le présent brevet,
- Optionnellement, un calculateur d'élasticité 43, utilisant les données de 42 pour obtenir la mesure souhaitée d'élasticité.

Optionnellement, le calculateur 41 peut également inclure la gestion d'une interface utilisateur 45, permettant l'intégration de l'ensemble des fonctions de calcul de l'appareil dans un très faible volume.

Le dispositif ainsi décrit permet notamment une intégration de l'ensemble des fonctions à proximité du transducteur lui-même. Ceci permet de limiter au maximum le transfert et le stockage intermédiaire de données. Ceci présente plusieurs avantages, notamment en termes de compacité globale du dispositif, de coût ou de qualité des signaux mesurés.

La présente invention est susceptible de variantes, notamment en effectuant diverses opérations, telle que la détermination du paramètre relatif au déplacement, au moyen de plus de deux lignes successives ou non.

Dans une variante, le dispositif comprend plusieurs transducteurs ou éléments de telle sorte que la ligne ultrasonore correspondant au tir i est une matrice formée par les données reçues à partir des différents groupes d'éléments en fonction du temps.

Dans une variante, le dispositif est associé à un transducteur tel que celui décrit dans la demande de brevet FR 0652140, déposée le 15 Juin 2006 par la société Echosens

Un tel dispositif traite plusieurs acquisitions réalisées simultanément sur plusieurs axes géométriques.

L'invention est décrite dans ce qui précède à titre d'exemple. Il est entendu que l'homme du métier est à même de réaliser différentes variantes du dispositif et du procédé pour la mesure de l'élasticité d'un organe humain ou animal, en particulier en associant ledit dispositif et/ou procédé à un dispositif et/ou procédé d'endoscopie, de laparoscopie, de biopsie ou tout autre dispositif ou procédé du type sans pour autant sortir du cadre du brevet.

**Revendications**

1. Dispositif (30, 50) destiné à mesurer des propriétés viscoélastiques de tissus biologiques grâce à un traitement d'ondes ultrasonores réfléchies par ces tissus lorsqu'ils sont parcourus par une onde de cisaillement, ce dispositif comprenant :

- Des moyens (M1, M2, M3) pour former des lignes (L1, L2, L3) de données telles que chaque ligne (L1, L2, L3) comprend des données relatives aux ondes ultrasonores réfléchies à partir d'un même tir (T1, T2, T3), et
- Des moyens (35) pour déterminer un paramètre relatif (3b) au déplacement entre les tissus et un transducteur émettant les tirs (T1, T2, T3),
- Des moyens (37) pour calculer un déplacement intrinsèque du milieu à partir d'un ensemble de lignes (L1, L2, L3) formant une acquisition (3a),

**caractérisé en ce qu'**il comprend des moyens (36) pour traiter de premières lignes (L1, L2) ultrasonores, à l'aide du paramètre relatif (3b), avant ou pendant la formation de secondes lignes (L3) ultrasonores de cette même acquisition (3a), afin de déterminer le déplacement intrinsèque (3d(L1)) des tissus biologiques à partir de ces premières lignes.

2. Dispositif (30, 50) selon la revendication 1 **caractérisé en ce qu'**il comprend des moyens pour calculer le déplacement intrinsèque (3d(L1)) pour un instant et à une profondeur donnés.

3. Dispositif (30, 50) selon la revendication 1 ou 2 **caractérisé en ce qu'**il comprend des moyens pour former avec différents déplacements intrinsèques (3d(L1)) un tableau (3D) chronologique tel que chaque colonne ou ligne du tableau (3D) est fonction de la profondeur à laquelle sont mesurés des déplacements intrinsèques (3d(L1)).

4. Dispositif (30, 50) selon la revendication 3 **caractérisé en ce qu'**il comprend des moyens pour que le temps de calcul ($\Delta$(cal1)) d'un déplacement intrinsèque (3d(L1)) soit inférieur au temps ($\Delta$(tir)) séparant deux tirs (T1, T2) ultrasonores successifs.

5. Dispositif (30, 50) selon la revendication 4 **caractérisé en ce qu'**il comprend des moyens pour que la mémoire (M1) allouée à une première ligne ultrasonore (L1) soit rendue disponible après son traitement pour mémoriser une seconde ligne ultrasonore (L3) ultérieure.

6. Dispositif (30, 50) selon la revendication 5 **caractérisé en ce qu'**il comprend des moyens pour traiter les ondes ultrasonores avec deux premières mémoires (M1, M2) dédiées à un calcul de déplacement intrinsèque, et une troisième mémoire (M3) stockant une ligne (L3) en cours d'acquisition.

7. Dispositif (30, 50) selon l'une des revendications précédentes **caractérisé en ce que** le même calculateur effectue de façon simultanée des opérations liées à une formation de ligne ultrasonore et à un calcul de déplacement intrinsèque.

8. Dispositif (30, 50) selon l'une des revendications précédentes **caractérisé en ce qu'**il comprend des moyens pour que les lignes ultrasonores (L1, L2, L3) proviennent d'un même élément émetteur/récepteur.

9. Dispositif (30, 50) selon l'une des revendications 1 à 8 **caractérisé en ce qu'**il comprend des moyens pour que des lignes ultrasonores soient formées grâce à une focalisation électronique avec au moins un transducteur comprenant plusieurs éléments.

10. Dispositif (30, 50) selon l'une des revendications précédentes **caractérisé en ce qu'**il comprend des moyens pour calculer un déplacement relatif du transducteur par rapport aux tissus observés à l'aide d'au moins :

- une mesure physique externe, telle que la position dudit transducteur par rapport à un référentiel donné,
- une mise en forme d'une mesure biophysique, tel qu'un signal obtenu à partir d'un rythme cardiaque ou respiratoire, ou
- un calcul effectué à partir des données ultrasonores.

11. Dispositif (30, 50) selon l'une des revendications précédentes **caractérisé en ce qu'**il comprend des moyens pour que le déplacement intrinsèque (3d(L1)) représente ou dérivé d'au moins un des éléments suivants : une mesure de déplacement, une vitesse, une vitesse de déformation, une mesure de déformation.

12. Dispositif (30, 50) selon l'une des revendications précédentes **caractérisé en ce qu'**il comprend des moyens pour traiter en parallèle différentes acquisitions réalisées sur différents axes géométriques.

13. Dispositif (30, 50) selon l'une des revendications précédentes **caractérisé en ce qu'**il comprend des moyens pour effectuer des mesures successives de façon continue.

14. Procédé destiné à mesurer des propriétés viscoélastiques de tissus biologiques grâce à un traitement d'ondes ultrasonores réfléchies par ces tissus lorsqu'ils sont parcourus par une onde de cisaillement, ce procédé étant **caractérisé en ce qu'**un dispositif conforme à l'une des revendications 1 à 13 étant utilisé, il comprend :

- L'étape d'utiliser des moyens (M1, M2, M3) pour former des lignes (L1, L2, L3) de données telles que chaque ligne (L1, L2, L3) comprend des données relatives aux ondes ultrasonores réfléchies à partir d'un même tir (T1, T2, T3),

- L'étape d'utiliser des moyens (35) pour déterminer un paramètre relatif (3b) au déplacement entre les tissus et un transducteur émettant les tirs (T1, T2, T3),

- L'étape d'utiliser des moyens (37) pour calculer un déplacement intrinsèque du milieu à partir d'un ensemble de lignes (L1, L2, L3) formant une acquisition (3a), et

- L'étape d'utiliser des moyens (36) pour traiter deux premières lignes (L1, L2) ultrasonores, à l'aide du paramètre relatif (3b), avant ou pendant la formation de secondes lignes (L3) ultrasonores de cette même acquisition (3a), afin de déterminer le déplacement intrinsèque (3d(L1)) des tissus biologiques à partir de ces premières lignes.

15. Sonde dédiée à la mesure de PV de tissus biologiques par élastographie, **caractérisé en ce qu'**elle comprend un dispositif conforme à l'une des revendications 1 à 13.

**Patentansprüche**

1. Vorrichtung (30, 50), die zur Messung der viskoelastischen Eigenschaften von biologischen Geweben dank einer Behandlung durch Ultraschallwellen bestimmt ist, die von diesen Geweben reflektiert werden, wenn sie durch eine Abscherwelle durchlaufen werden, wobei diese Vorrichtung umfasst:

    - Mittel (M1, M2, M3) zur Bildung von Datenlinien (L1, L2, L3) derart, dass jede Linie (L1, L2, L3) Daten bezüglich der von einem und demselben Schuss (T1, T 2, T3) reflektierten Ultraschallwellen umfasst und
    - Mittel (35) zur Bestimmung eines Parameters (3b) bezüglich der Verschiebung zwischen den Geweben und einem die Schüsse (T1, T2, T3) ausgebenden Transduktor,
    - Mittel (37) zur Berechnung einer intrinsischen Verschiebung der Mitte ab einer Gruppe von Linien (L1, L2, L3), die einen Erwerb (3a) bilden,

**dadurch gekennzeichnet, dass** sie Mittel (36) zur Bearbeitung von ersten Ultraschalllinien (L1, L2) mithilfe des ersten relativen Parameters (3b) vor oder während der Bildung von zweiten Ultraschalllinien (L3) dieses selben Erwerbs (3a) umfasst, um die intrinsische Verschiebung (3d(L1)) der biologischen Gewebe ab diesen ersten Linien zu bestimmen.

2. Vorrichtung (30, 50) gemäß Anspruch 1, **dadurch gekennzeichnet, dass** sie Mittel zur Berechnung der intrinsischen Verschiebung (3d(L1)) für einen bestimmten Moment und eine bestimmte Tiefe umfasst.

3. Vorrichtung (30, 50) gemäß Anspruch 1 oder 2, **dadurch gekennzeichnet, dass** sie Mittel zur Bildung einer chronologischen Tabelle (3d) mit unterschiedlichen intrinsischen Verschiebungen (3d(L1)) derart umfasst, dass jede Spalte oder Zeile der Tabelle (3d) von der Tiefe abhängt, mit der die intrinsischen Verschiebungen (3d(L1)) gemessen werden.

4. Vorrichtung (30, 50) gemäß Anspruch 3, **dadurch gekennzeichnet, dass** sie Mittel umfasst, damit die Berechnungszeit ($\Delta$(cal1)) einer intrinsischen Verschiebung (3d(L1)) kürzer ist als die Zeit ($\Delta$(tir)), die zwei sukzessive Ultraschall-Schüsse (T1, T2) voneinander trennt.

5. Vorrichtung (30, 50) gemäß Anspruch 4, **dadurch gekennzeichnet, dass** sie Mittel umfasst, damit der einer ersten Ultraschalllinie (L1) zugeordnete Speicher nach seiner Bearbeitung zur Speicherung einer zweiten, späteren Ultraschalllinie (L3) verfügbar gemacht wird.

6. Vorrichtung (30, 50) gemäß Anspruch 5, **dadurch gekennzeichnet, dass** sie Mittel zur Bearbeitung der Ultraschallwellen mit zwei ersten Speichern (M1, M2) umfasst, die für eine Berechnung der intrinsischen Verschiebung dediziert sind, und einen dritten Speicher (M3), der eine sich im Erwerb befindliche Linie (L3) speichert.

7. Vorrichtung (30, 50) gemäß einem der voranstehenden Ansprüche, **dadurch gekennzeichnet, dass** derselbe Rechner gleichzeitig mit der Bildung einer Ultraschallinie und einer Berechnung einer intrinsischen Verschiebung verbundene Operationen ausführt.

8. Vorrichtung (30, 50) gemäß einem der voranstehenden Ansprüche, **dadurch gekennzeichnet, dass** sie Mittel umfasst, damit die Ultraschalllinien (L1, L2, L3) von einem und demselben Sender- / Empfängerelement stammen.

9. Vorrichtung (30, 50) gemäß Anspruch 1 bis 8, **dadurch gekennzeichnet, dass** sie Mittel umfasst, damit Ultraschalllinien dank einer elektronischen Fokalisierung mit wenigstens einem mehrere Elemente umfassenden Transduktor gebildet sind.

10. Vorrichtung (30, 50) gemäß einem der voranstehenden Ansprüche, **dadurch gekennzeichnet, dass** sie Mittel umfasst, um eine relative Verschiebung des Transduktors im Verhältnis zu den beobachteten Geweben zu berechnen, mithilfe von wenigstens:

- einer externen physikalischen Messung, wie z. B. der Position des genannten Transduktors im Verhältnis zu einer bestimmten Bezugsgröße,
- einer Formgebung einer biophysikalischen Messung, wie z. B. eines ausgehend von einem Herz- oder Atemrhythmus erhaltenen Signals oder
- einer ausgehend von Ultraschalldaten durchgeführten Berechnung.

**11.** Vorrichtung (30, 50) gemäß einem der voranstehenden Ansprüche, **dadurch gekennzeichnet, dass** sie Mittel umfasst, damit die intrinsische Verschiebung (3d(L1) wenigstens eines der folgenden Elemente darstellt oder ableitet: eine Verschiebungsmessung, eine Geschwindigkeit, eine Verformungsgeschwindigkeit, eine Verformungsmessung.

**12.** Vorrichtung (30, 50) gemäß einem der voranstehenden Ansprüche, **dadurch gekennzeichnet, dass** sie Mittel umfasst, um parallel unterschiedliche, auf unterschiedlichen geometrischen Achsen realisierte Erwerbe zu bearbeiten.

**13.** Vorrichtung (30, 50) gemäß einem der voranstehenden Ansprüche, **dadurch gekennzeichnet, dass** sie Mittel umfasst, um kontinuierlich sukzessive Messungen durchzuführen.

**14.** Vorrichtung, die zur Messung der viskoelastischen Eigenschaften von biologischen Geweben dank einer Behandlung von Ultraschallwellen bestimmt ist, die durch diese Gewebe reflektiert werden, wenn sie durch eine Abscherwelle durchlaufen werden, wobei dieses Verfahren **dadurch gekennzeichnet, ist, dass** eine Anspruch 1 bis 13 entsprechende Vorrichtung verwendet wird, sie umfasst:

- die Stufe der Nutzung der Mittel (M1, M2, M3) zur Bildung der Datenlinien (L1, L2, L3) derart, dass jede Linie (L1, L2, L3) Daten bezüglich der Ultraschallwellen umfasst, die ausgehend von einem und demselben Schuss (T1, T2, T3) reflektiert werden,
- die Stufe der Nutzung der Mittel (35) zur Bestimmung eines Parameters (3b) bezüglich der Verschiebung zwischen den Geweben und einem die Schüsse (T1, T2, T3) ausgebenden Transduktor,
- die Stufe der Nutzung der Mittel (37) zur Berechnung einer intrinsischen Verschiebung der Mittel ausgehend von einer Gruppe von Linien (L1, L2, L3), die einen Erwerb (3a) darstellen, und

- die Stufe der Nutzung der Mittel (36) zur Bearbeitung von zwei ersten Ultraschallinien (L1, L2) mithilfe des relativen Parameters (3b) vor oder während der Bildung von zweiten Ultraschallinien (L3) dieses selben Erwerbs (3a), um die intrinsische Verschiebung (3d(L1)) der biologischen Gewebe ausgehend von diesen ersten Linien zu bestimmen.

**15.** Für die Messung von viskoelastischen Eigenschaften von biologischen Geweben per Elastographie bestimmte Sonde, **dadurch gekennzeichnet, dass** sie eine Vorrichtung gemäß Anspruch 1 bis 13 umfasst.

**Claims**

**1.** Device (30, 50) for measuring the viscoelastic properties of biological tissues using a processing of ultrasound waves reflected by said tissues when a shear wave runs across them, wherein said device comprises:

- Means (M1, M2, M3) for forming lines (L1, L2, L3) of data such that each line (L1, L2, L3) comprises data relative to the reflected ultrasound waves from a same shot (T1, T2, T3), and
- Means (35) for determining a parameter (3b) relative to the movement between the tissues and a transducer emitting the shots (T1, T2, T3),
- Means (37) for calculating an intrinsic movement of the medium from a set of lines (L1, L2, L3) defining an acquisition (3a),

**characterised in that** it comprises means (36) for processing the first ultrasound lines (L1, L2), using the relative parameter (3b), before or during the formation of the second ultrasound lines (L3) of this same acquisition (3a), in order to determine the intrinsic movement (3d(L1)) of the biological tissues from said first lines.

**2.** Device (30, 50) according to claim 1 **characterised in that** it comprises means for calculating the intrinsic movement (3d(L1)) for a given instant and at a given depth.

**3.** Device (30, 50) according to claim 1 or 2 **characterised in that** it comprises means for forming with different intrinsic movements (3d(L1)) a chronological table (3D) such that each column or line of the table (3D) is a function of the depth at which the intrinsic movements (3d(L1)) are measured.

**4.** Device (30, 50) according to claim 3 **characterised in that** it comprises means so that the calculation time ($\Delta$(cal1)) of an intrinsic movement (3d(L1)) is less than the time ($\Delta$(shot)) separating two successive ultrasound shots (T1, T2).

5. Device (30, 50) according to claim 4 **characterised in that** it comprises means so that the memory (M1) allocated to a first ultrasound line (L1) is made available after its processing for memorising a second later ultrasound line (L3).

6. Device (30, 50) according to claim 5 **characterised in that** it comprises means for processing the ultrasound waves with two first memories (M1, M2) dedicated to a calculation of intrinsic movement, and a third memory (M3) storing a line (L3) during acquisition.

7. Device (30, 50) according to one of the preceding claims **characterised in that** the same calculator carries out simultaneously the operations linked to a formation of ultrasound line and to a calculation of intrinsic movement.

8. Device (30, 50) according to one of the preceding claims **characterised in that** it comprises means so that the ultrasound lines (L1, L2, L3) come from a same transmitter/receiver element.

9. Device (30, 50) according to one of claims 1 to 8 **characterised in that** it comprises means so that ultrasound lines are formed using an electronic focusing with at least one transducer including several elements.

10. Device (30, 50) according to one of the preceding claims **characterised in that** it comprises means for calculating a relative movement of the transducer compared to the tissues observed using at least:

   - an external physical measurement, such as the position of said transducer compared to a given referential,
   - a formatting of a biophysical measurement, such as a signal obtained from a cardiac or respiratory rhythm, or
   - a calculation carried out from ultrasound data.

11. Device (30, 50) according to one of the preceding claims **characterised in that** it comprises means so that the intrinsic movement (3d(L1)) represents or derives from at least one of the following elements: a movement measurement, a speed, a deformation speed, a deformation measurement.

12. Device (30, 50) according to one of the preceding claims **characterised in that** it comprises means for processing in parallel different acquisitions carried out on different geometric axes.

13. Device (30, 50) according to one of the preceding claims **characterised in that** it comprises means for carrying out successive measurements in a continuous manner.

14. Method for measuring the viscoelastic properties of biological tissues using a processing of ultrasound waves reflected by said tissues when a shear wave runs across them, said method being **characterised in that** a device according to one of claims 1 to 13 being used, it comprises:

   - The step of using means (M1, M2, M3) for forming lines (L1, L2, L3) of data such that each line (L1, L2, L3) comprises data relative to the reflected ultrasound waves from a same shot (T1, T2, T3),
   - The step of using means (35) for determining a parameter (3b) relative to the movement between the tissues and a transducer emitting the shots (T1, T2, T3),
   - The step of using means (37) for calculating an intrinsic movement of the medium from a set of lines (L1, L2, L3) defining an acquisition (3a), and
   - The step of using means (36) for processing two first ultrasound lines (L1, L2), using the relative parameter (3b), before or during the formation of second ultrasound lines (L3) of this same acquisition (3a), in order to determine the intrinsic movement (3d(L1)) of biological tissues from said first lines.

15. Probe for measuring the VP of biological tissues by elastography, **characterised in that** it comprises a device according to one of claims 1 to 13.

M1   M2   M3   M4   M5        M n

| L1 | L2 | L3 | L4 | L5 | | L n |

1A

2

5

1A
1B

4

1A          1B

1C          6

7

1C   *   1C

1D

10

3

1D   8

FIG.1

FIG.2

FIG.3

FIG.4a

FIG.4b

FIG. 5

**EP 2 129 296 B1**

## RÉFÉRENCES CITÉES DANS LA DESCRIPTION

**Documents brevets cités dans la description**

- FR 2869521

- FR 0652140